# EUROPÄISCHE PATENTANMELDUNG

(11) **EP 4 339 787 A1**
(43) Veröffentlichungstag der Anmeldung: **20.03.2024**
(21) Anmeldenummer: 22195302.9
(22) Anmeldetag: 13.09.2022
(51) Int. Cl.: G06F 11/36

(54) **TEST VON LEGACY-CODE ZUR GERÄTESTEUERUNG VON MEDIZINISCHEN GERÄTEN**

(71) Anmelder: Fresenius Medical Care Deutschland GmbH, 61352 Bad Homburg (DE)
(72) Erfinder: SCHÄFER, Ingo, 65760 Eschborn (DE)
(74) Vertreter: Schwarz, Claudia

(57) **Zusammenfassung**

Die vorliegende Erfindung betrifft das Testen von Softwarecode eines Produktivsystems für ein medizintechnisches Gerät, wie z.B. ein Dialysegerät oder ein Blutbehandlungsgerät, wenn hardwareseitige und/oder softwareseitige Änderungen an dem Produktivsystem vorgenommen werden. Der Softwarecode kann als Legacy-Code ausgebildet sein. Beim Betreiben des Produktivsystems mit dem Legacy-Code werden Felddaten gesammelt, die bei dem Test zumindest indirekt berücksichtigt werden.

## Beschreibung

Die Erfindung betrifft eine Funktionalitätsprüfung von Softwarecode, insbesondere von Legacy-Code, der zum Steuern und/oder Betreiben eines Produktivsystems für ein medizintechnisches Gerät, insbesondere ein Dialysegerät, verwendet wird. Das Produktivsystem umfasst Hardware, auf der der zu prüfende Softwarecode installiert ist. Bei Änderungen am Produktivsystem muss der Softwarecode einer Funktionalitätsprüfung, insbesondere auf spezifikationskonforme Funktionalität, unterzogen werden. Die Erfindung bezieht sich insbesondere auf ein Verfahren zur Funktionalitätsprüfung, auf ein Testsystem, auf ein medizinisches Gerät und ein medizinisches Behandlungssystem sowie ein Computerprogramm.

Software zur Steuerung komplexer, insbesondere medizintechnischer, Geräte und Systeme basiert heute vielfach auf veraltetem, monolithischem Programmcode (Legacy-Code), welcher über Jahrzehnte kontinuierlich weiterentwickelt worden ist. Nachteilig hierbei ist, dass sich aktuelle Programmierstandards sowie regulatorische Erfordernisse im Laufe der Zeit ändern. Insbesondere im medizinischen Sektor steigen die Anforderungen im Bereich Softwaresicherheit kontinuierlich. Ein Beispiel hierfür ist die Anforderung, aktuelle Softwaretests, wie beispielsweise Unit-Tests, zur Verfügung zu stellen, um zulassungs- und sicherheitsrelevante Regulatorien zu erfüllen, damit die Gerätesoftware sowie das zugrundeliegende medizinische Gerät weiterhin betrieben werden können.

Legacy-Code kann jedoch in vielen Fällen nur unter hohem Aufwand modernen Softwaretests unterzogen werden. Aufgrund veralteter Software- und Hardwarestrukturen ist es beispielsweise in der Regel technisch nur bedingt möglich, Unit-Tests durchzuführen bzw. aussagekräftige Resultate hieraus zu generieren. Zum Beispiel werden aufgrund veralteter Legacy-Code-Segmente angebliche Fehler ermittelt, welche sich bei genauerer Analyse und Betrachtung als falsch-positive Resultate herausstellen. Präzise Analysen einzelner Resultate sind jedoch bei umfangreichem Programmcode, der sich beispielsweise in einer Vielzahl von Medizintechnikgeräten finden lässt, in der Regel unmöglich. Aufgrund positiver Felderfahrungen mit Legacy-Systemen, also der Kombination aus einer ursprünglichen Hardwareimplementierung und einer verwendeten Systemsoftware basierend auf Legacy-Code, ist dieses Problem jedoch zunächst nur bedingt kritisch. Der Grund hierfür ist eine empirische Überprüfung sog. Legacy-Systeme (d.h. Legacy-Softwarecode implementiert auf einer Hardware) auf Basis einer dokumentierten jahrelangen und fehlerfreien Betriebshistorie. Bei Änderung z.B. einzelner Hardwarekomponenten des Legacy-Systems, z.B. des Hauptprozessors oder der Hauptplatine, kann jedoch eine erneute regulatorische Überprüfung erforderlich werden. Zur Sicherstellung der Patientensicherheit ist dies meistens sogar unumgänglich, da sich die positive Felderfahrung lediglich auf die ursprüngliche Kombination aus Hardwareimplementierung und Systemsoftware anwenden lässt und eine Kombination aus neuer Hardwareimplementierung sowie veralteter Systemsoftware zu potenziell unvorhersehbarem Systemverhalten führen kann. Eine vergleichbare Situation stellt sich analog im Falle von Softwareänderungen bei gleichzeitiger Beibehaltung einer ursprünglichen Hardwareimplementierung. Die begrenzte Anwendbarkeit moderner Softwaretestverfahren zu Qualitätssicherungszwecken im Falle von Legacy-Code bzw. von Legacy-Systemen, stellt folglich ein technisch bislang nur unzureichend gelöstes Problem dar.

Aufgabe der vorliegenden Erfindung ist es deshalb, eine Überprüfung oder ein Testen der Funktionalität von Softwarecode bei Änderungen an einem rechnergestützten Produktivsystem zu ermöglichen oder zu verbessern. Die Überprüfung soll insbesondere auch dann möglich sein, wenn der Softwarecode als Legacy-Code ausgebildet ist.

Zur Lösung dieser Aufgabe schlägt die Erfindung ein computer-implementiertes Verfahren, ein Computerprogramm, ein Testsystem, ein medizinisches Gerät mit einem solchen Testsystem und ein medizinisches Behandlungssystem gemäß den beiliegenden Patentansprüchen vor.

Im Folgenden wird die Lösung der Aufgabe anhand des computer-implementierten Verfahrens beschrieben. Dabei erwähnte Merkmale, Vorteile und/oder alternative Ausführungsformen sind ebenso auch auf die anderen beanspruchten Gegenstände zu übertragen und umgekehrt. Mit anderen Worten können auch die gegenständlichen Ansprüche (die beispielsweise auf ein Gerät, ein System oder auf ein Computerprogramm gerichtet sind) mit den Merkmalen weitergebildet sein, die in Zusammenhang mit dem Verfahren beschrieben oder beansprucht sind und umgekehrt. Die entsprechenden funktionalen Merkmale des Verfahrens werden dabei durch entsprechende gegenständliche Module, insbesondere durch Hardware-Module oder Mikroprozessor-Module, des Systems bzw. des Produktes ausgebildet und umgekehrt. Die vorstehend im Zusammenhang mit dem Verfahren beschriebenen bevorzugten Ausführungsform der Erfindung werden nicht explizit für die Vorrichtung wiederholt. Im Allgemeinen sind in der Informatik eine Software-Implementierung und eine entsprechende Hardware-Implementierung (z. B. als eingebettetes System) gleichwertig. So kann z. B. ein Verfahrensschritt zum "Ausgeben" eines Testergebnisses mit einer Ausgabeschnittstelle und den Testergebnissen durchgeführt werden. Um Redundanz zu vermeiden, wird die Vorrichtung deshalb nicht noch einmal explizit beschrieben, obwohl es auch in den alternativen Ausführungsformen, die in Bezug auf das Verfahren beschrieben sind, verwendet werden kann.

Gemäß einem ersten Aspekt bezieht sich die Erfindung auf ein computer-implementiertes Verfahren zur Funktionalitätsprüfung von Softwarecode eines Produktivsystems. Das Produktivsystem kann zur Steuerung und/oder zum Betrieb eines medizintechnischen Gerätes ausgebildet sein. Das Produktivsystem kann für das medizintechnische Gerät betrieben werden. Die Funktionalitätsprüfung des Softwarecodes soll insbesondere ausgeführt werden, wenn das Produktivsystem durch Änderungen an dessen Hardware und/oder an dessen Softwarecode geändert wird und dadurch ein geändertes System erzeugt wird. Das geänderte System und das (bisherige) Produktivsystem können für eine konfigurierbare Zeitphase parallel existieren. Das Verfahren kann die folgenden Verfahrensschritte umfassen:
- Ausführen eines Testverfahrens auf dem Produktivsystem und Erzeugen eines ersten Testergebnisses;
- Ausführen des Testverfahrens auf dem geänderten System und Erzeugen eines zweiten Testergebnisses;
- Vergleichen des ersten Testergebnisses mit dem zweiten Testergebnis, um eine spezifikationskonforme Funktionalität des geänderten Systems zu prüfen;
- Ausgeben eines Testergebnisses.

In einer ersten Variante kann das Testverfahren - sozusagen direkt - auf dem Produktivsystem und/oder dem geänderten System ausgeführt werden. In einer zweiten Variante enthält das Testverfahren die Ausführung einer Testsuite auf dem Produktivsystem und/oder dem geänderten System.

Im Folgenden werden die Begrifflichkeiten der Anmeldung näher erläutert.

Das Vergleichen des ersten Testergebnisses mit dem zweiten Testergebnis liefert das Testergebnis, welches dann final ist und/oder ausgegeben werden kann.

Das medizintechnische Gerät ist eine technische Einrichtung für medizinische Zwecke und/oder medizinische Behandlungen. Es umfasst medizinische Einheiten und rechnerbasierte bzw. elektronische Einheiten, die im Wesentlichen der Steuerung und/oder dem Betrieb des medizinischen Geräts dienen. In einer bevorzugten Ausführungsform ist das medizinische Gerät ein Gerät zur Blutbehandlung, insbesondere ein Dialysegerät. Das medizinische Gerät kann auch ein Gerät für die Peritonealdialyse oder ein Plasmapheresegerät sein. Die Dialyse wird üblicherweise eingesetzt, um die Nierenfunktion zu ersetzen, indem Abfallgifte und überschüssiges Wasser entfernt werden. Während der Behandlung und damit auch während des Betriebs des medizinischen Geräts muss der korrekte Betrieb sichergestellt werden. Der korrekte Betrieb des medizintechnischen Gerätes erfordert eine korrekte Funktionalität des Softwarecodes, der auf dem Produktivsystem implementiert ist und einer Überprüfung unterzogen werden muss.

Das Produktivsystem ist oder umfasst ein rechnerbasiertes System. Das Produktivsystem kann auf einem medizintechnischen Gerät betrieben werden oder für ein medizintechnisches Gerät (z.B. als Serverlösung) betrieben werden. Das Produktivsystem kann somit zumindest teilweise direkt auf dem medizintechnischen Gerät ausgebildet sein; alternativ oder kumulativ kann das Produktivsystem zumindest teilweise auf einem anderen Rechnersystem (als das Produktivsystem, z.B. in Form einer Cloud-basierten Lösung) ausgebildet sein, das über eine entsprechende Schnittstellenanbindung an das Produktivsystem und/oder das medizintechnische Gerät angebunden ist. Das Produktivsystem kann eine elektronische Einheit umfassen. Die elektronische Einheit kann Hardware, insbesondere unterschiedliche Hardwarekomponenten, wie z.B. eine CPU, einen Speicher und/oder physikalische Schnittstellen etc. und/oder einen Softwarecode umfassen.

Die elektronische Einheit kann ein Rechensystem (z.B. in Form einer elektronischen Schaltung, wie z.B. einem FPGA, oder einem embedded system, einem system-on-chip, SOC, oder in Form von einem IC-Baustein) umfassen mit einer beliebigen Kombination aus Hard- und Softwarekomponenten. Beispiele hierfür können eine Kombination aus einer gegebenen Hardwareimplementierung (z.B. Hauptrecheneinheit, Speicher und Hauptplatine) sein, welche sich auf Basis einer sich auf einem flüchtigen/nicht-flüchtigen Speicher befindlichen Software steuern lässt. Beispielsweise können Rechensysteme als Teil eines Behandlungssystems (z.B. ein Infusions- oder Dialysesystem) verwendet werden, um einzelne Komponenten des Behandlungssystems (Sensoren, Pumpen, etc.) zu steuern.

Das Testverfahren für das Rechensystem kann prinzipiell alle Typen gängiger Softwaretestverfahren umfassen, welche dazu geeignet sind, eine spezifikationskonforme Funktionalität zwischen Software und Hardwareimplementierung zu gewährleisten. Beispiele hierfür sind Unit-Tests, welche einzelne Programmabschnitte ("Units") innerhalb eines Programmcodes, z.B. Funktionen oder Klassen, testen. Üblicherweise werden hierzu zunächst sog. "Mocks" erzeugt, welche eine eigenständige Lauffähigkeit einzelner Programmabschnitte erlauben.

Der eigentliche Programmtest wird auf Basis der identifizierten Units, der generierten Mocks sowie der entsprechenden Test-Inputs durchgeführt. Zur Erzeugung der Test-Inputs wird hierbei auf eine "symbolischen Simulation" zurückgegriffen, wobei hierbei die zu testende Unit in einen logischen Ausdruck überführt wird. Anschließend wird der mögliche Eingangswertebereich der zu testenden Unit überprüft, indem das Verhalten der betreffenden Unit kontrolliert wird. Der Programmtest ermöglicht somit, dass eben jene Units identifiziert werden können, welche beispielsweise aufgrund eines Fehlers ein unvorhergesehenes Verhalten (z.B. ein Systemabsturz des Rechensystems) zeigen können.

Bei dem Softwarecode kann es sich um einen sogenannte Legacy-Code handeln. Er kann als monolithischer Block implementiert sein. Ein Legacy-Softwarecode ist ein "alter" (nicht neu progammierter und schon länger im Feldeinsatz befindlicher) Code, der schon lange in den Geräten bzw. Produktivsystemen betrieben wurde und betriebsnotwendig an eine bestimmte Version eines Betriebssystems und/oder Hardwaremodells gebunden ist. Die Legacy-Software kann in einer alten Programmiersprache geschrieben sein und z.B. auf Mainframes liegen. Der Softwarecode kann in einer beliebigen Programmiersprache vorliegen und gespeichert sein. Der Softwarecode kann z.B. Maschinencode (z.B. in Assembler) sein.

Ein Softwarecodeabschnitt ist ein Teil bzw. ein Abschnitt des Softwarecodes. Der Softwarecodeabschnitt kann ein extrahiertes Codemodul sein. Die Extraktion erfolgt automatisch mittels eines Zerlegealgorithmus nach konfigurierbaren Zerlegekriterien.

Das Produktivsystem und insbesondere dessen Hardware und/oder dessen Softwarecode unterliegt Änderungen. Durch diese Änderungen entsteht ein geändertes System. Das geänderte System ist ebenfalls ein rechnerbasiertes System. Das geänderte System basiert quasi auf dem Produktivsystem und umfasst zusätzlich die Änderungen an der Hardware und/oder an dem Softwarecode. Das Produktivsystem und das geänderte System können zumindest über einen konfigurierbaren Zeitraum, insbesondere in dem das Verfahren zur Funktionalitätsprüfung ausgeführt wird, parallel existieren. In der Regel ist es z.B. notwendig, eine veraltete Hardware durch neuere Hardware zu ersetzen. Durch den Ersatz der "alten" Hardware durch die "neue" Hardware in Kombination mit dem "alten" (d.h. ursprünglichen) Softwarecode entsteht ein geändertes System. Das geänderte System und insbesondere dessen Softwarecode muss getestet werden, insbesondere auf spezifikationskonforme Funktionalität. Alternativ kann das geänderte System entstehen, indem die "alte" Hardware beibehalten wird (unverändert), aber der darauf deployte Softwarecode vollständig oder teilweise geändert wird.

Die Funktionalitätsprüfung ist eine Prüfung des Softwarecodes oder dessen Abschnitten, insbesondere Units. Der Softwarecode oder dessen Abschnitte werden dahingehend überprüft, ob die bereitgestellte Funktionalität auch derjenigen entspricht, die in einer Spezifikation festgelegt worden ist.

Das Testverfahren ist ein Teil der Funktionalitätsprüfung. Das Testverfahren testet die Softwarecodeabschnitte, insbesondere sogenannte Units, auf fehlerfreie Funktionalität. Das Testverfahren testet die Softwarecodeabschnitte insbesondere gegenüber einer vorgegebenen Spezifikation für die Software. Das Testverfahren kann erfindungsgemäß wiederholt und insbesondere zweimal auf unterschiedlichen Rechner-basierten Systemen angewendet werden. Eine erste Anwendung des Testverfahren testet oder prüft das (Laufzeit-) Verhalten des compilierten Softwarecodeabschnittes auf dem alten, unveränderten Produktivsystem und eine zweite Anwendung desselben Testverfahrens testet oder prüft das (Laufzeit-)Verhalten des compilierten Softwarecodeabschnittes auf dem geänderten System. Das Testverfahren kann dazu bestimmt sein, das Verhalten der Softwarecodeabschnittes mit dem SOLL-Verhalten (vorgegeben z.B. durch eine Spezifikation) oder gegenüber einem erwarteten Verhalten (z.B. gemäß dem Verhalten auf dem unveränderten Produktivsystem) zu vergleichen.

Für das Altsystem, also insbesondere das Legacy-System existieren in der Regel eine große Menge von Felddaten, die einen fehlerfreien Betrieb kennzeichnen. Diese Daten können in ihrer Gesamtheit auch als "Felderfahrung" bezeichnet werden. Eine positive Felderfahrung umfasst insbesondere, dass die im Feld generierten Eingangsdaten zu spezifikationskonformen Ausgangsdaten führen. Die Felderfahrung fließt vorteilhafterweise und/oder insbesondere indirekt bei der erfindungsgemäßen Funktionalitätsprüfung ein und/oder kann dort berücksichtigt werden. Dies erfolgt, indem das Testverfahren als erstes Testverfahren zunächst auf dem Produktivsystem ausgeführt wird, das quasi als Referenz- oder Benchmarksystem dient und von dem Kenntnis besteht, dass es in der Vergangenheit seit Längerem fehlerfrei funktioniert hat. Das erste Testergebnis repräsentiert somit per definitionem einen fehlerfreien Betrieb. Das erste Testergebnis repräsentiert insbesondere selbst dann einen fehlerfreien Betrieb, wenn das erste Testergebnis eine Menge von Fehlern liefert (dies kann z.B. dann der sein, wenn die Fehler nicht relevant sind oder innerhalb eines Toleranzbereichs für das medizintechnische Gerät liegen). Bei dem Testverfahren werden Eingangsdaten als Testdaten dem jeweiligen Softwarecodeabschnitt zugeführt und mit den Ausgabe- oder Ergebnisdaten abgeglichen.

Das Produktivsystem (mit dem Legacy-Softwarecode) befindet sich bereits seit längerer Zeit fehlerfrei im Betrieb, so dass also Felddaten gesammelt werden konnten, die einen fehlerfreien Betrieb repräsentieren.

Bei den Felddaten kann es sich um Sensordaten handeln, die während des Produktivsystembetriebs erfasst werden und die es ermöglichen, Rückschlüsse auf eine spezifikationskonforme Funktionalität des jeweiligen Rechensystems (Produktivsystem oder geändertes System) zuzulassen. Die Felddaten können automatisch über Sensoren erfasst werden. Beispielsweise kann dies eine Fehlerrate und/oder Fehlerreports eines Rechensystem innerhalb eines bestimmten Zeitraums, z.B. innerhalb mehrerer Jahre, umfassen. Eine spezifikationskonforme Funktionalität des Rechensystems wäre beispielsweise dann gegeben, wenn die Fehlerrate bestimmte regulatorische Grenzwerte nicht überschreitet und/oder die übermittelten Fehlerzahlen und/oder Fehlertypen, verhältnismäßig wenige Fehler aufweisen, wobei diese nur eine geringe Sicherheitsrelevanz aufweisen, d.h. den regulatorischen Anforderungen (z.B. IEC 61508 oder IEC 62304 in der Medizintechnik) entsprechen. In diesem Zusammenhang könnte eine spezifikationskonforme Funktionalität des Rechensystems auch dann gegeben sein, wenn in einem festgelegten Zeitraum keine sicherheitskritischen Fehler aufgezeichnet werden konnten.

In einer vorteilhaften Ausführungsform der Erfindung ist oder umfasst das Testverfahren einen Unittest. Ein Unittest ist ein Test oder eine Prüfung im Softwareentwicklungsprozess, bei dem die kleinsten prüfbaren Teile oder Einheiten einer Anwendung, die so genannten Units, einzeln und unabhängig voneinander auf ihren ordnungsgemäßen oder spezifikationsgemäßen Betrieb hin untersucht werden. Für weitere Details des an sich bekannten Ansatzes sei auf die Veröffentlichung Hamill, Paul (2004). Unit Test Frameworks: Tools for High-Quality Software Development. O'Reilly Media, Inc. ISBN 9780596552817 verwiesen. Alternativ kann das Testverfahren als Regressionstest ausgebildet sein, der dazu dient, Testfälle zu wiederholen. Die Erfindung ermöglicht die Prüfung eines veränderten Systems im Vergleich zu einem Altsystem.

Eine Verwendung des erfindungsgemäßen Verfahrens bzw. Testsystems bzw. eine Nutzung der Erfindung auch auf höheren Testebenen ist im Prinzip möglich (wie z.B. für Integrationstests, Systemtests). Das ist aber nur dann vorteilhaft, wenn dadurch andere Aspekte (z. B. Synchronisation) in die Testergebnisse einfließen, die in den detaillierteren Ebenen entfallen.

Das Testergebnis kann im einfachsten Fall ein "bestanden/nicht-bestanden" und damit ein binäres Ergebnis sein. Alternativ kann es noch weitere Metadaten umfassen, wie z.B. Angaben über eine Testabdeckung. Es kann auch Schwellenwerte angeben, insbesondere einen Grad der Übereinstimmung zwischen dem ersten und zweiten Testergebnis. In einer Konfigurationsphase kann konfiguriert werden, ob der Test nur dann als "erfolgreich absolviert" bzw. "bestanden" gilt, wenn eine 100% Übereinstimmung zwischen ersten und zweiten Testergebnis vorliegt oder ob auch eine geringere Übereinstimmung als erfolgreich gelten soll, z.B. in einem Bereich von 80-100%, insbesondere zwischen 90% und 100%. Eine Unschärfe (keine 100% Übereinstimmung) kommt insbesondere für pseudo-analoge Größen (z. B. Rundungsdifferenzen bei reellen Zahlen) oder nicht-funktionalen Parametern in Frage (z.B. Rechenzeitbedarf, Speicherplatzbedarf, Reaktionszeiten etc.).

In einer weiteren vorteilhaften Ausführungsform der Erfindung umfasst das Verfahren zumindest einen der folgenden Schritte:
- Automatisches Zerlegen des Softwarecodes in Softwarecodeabschnitte;
- Automatisches Erstellen jeweils einer Testumgebung für jeweils einen Softwarecodeabschnitt aller Softwarecodeabschnitte und/oder
- Automatisches Erstellen von Teststimuli bzw. Eingangsdaten zur Ausführung des Testverfahrens. (Testergebnisse entstehen bei oder nach Ausführung des Tests und repräsentieren auch Testdaten).

Das spezifische Erstellen einer Testumgebung für jeweils einen Softwarecodeabschnitt hat den Vorteil, dass die Testumgebung zielgerichtet und/oder zugeschnitten auf den Softwarecodeabschnitt und/oder dessen Funktionalität erzeugt werden kann. Weiterhin hält eine minimale Testumgebung den Aufwand (z.B. Rechenzeit, Speicherplatz) bei Stimuli-Generierung und/oder Testausführung in Grenzen. Üblicherweise entstehen zu jedem Abschnitt sehr viele Tests.

Eine Testumgebung ist eine computerbasierte Umgebung oder Grundlage für die Durchführung des anschließenden Tests. Die Testumgebung enthält insbesondere Teile, die Voraussetzung für das Ausführen des Testobjekts sind, die aber nicht Teil des Testobjekts selbst sind. Beispiele sind globale Daten oder Funktionen.

Das Erstellen der Testumgebung erfolgt vorzugsweise automatisch. "Automatisch" soll in dieser Anmeldung verstanden werden als "ohne Benutzerinteraktion". Alternativ kann das Erstellen der Testumgebung auch halbautomatisch erfolgen durch entsprechende Konfigurationen oder Einstellungen des Anwenders.

In einer vorteilhaften Ausführungsform der Erfindung erfolgt ein Zerlegen des Softwarecodes in Softwarecodeabschnitte automatisch. Insbesondere durch Anwendung von formalen vom Anwender auswählbaren und/oder konfigurierbaren Zerlegekriterien.

Die Zerlegekriterien können eine Dateizugehörigkeit, eine Namensgebung und/oder syntaktische und/oder semantische Eigenschaften des Softwarecodes und/oder der Softwarecodeabschnitt umfassen. Beispielsweise wird bei dem Zerlegekriterium der "Dateizugehörigkeit" der Softwarecode z.B. Zeile für Zeile überprüft, ob er zur selben Datei gehört. Solange die ausgewerteten Zeilen zur selben Datei gehören, handelt es sich um denselben Softwarecodeabschnitt; sobald die Zeile aber zu einer anderen Datei gehört, ist das Ende des Softwarecodeabschnittes erreicht und ein neuer Softwarecodeabschnitt wird erzeugt. Ebenso können andere syntaktische und/oder semantische Eigenschaften des Softwarecodes als Zerlegekriterium ausgewertet werden. Dabei kann es sich z.B. um Funktionsgrenzen, Schleifenkörper, Blockgrenzen handeln. Alternativ oder kumulativ kann auch eine vorkonfigurierbare Zeilenanzahl des Softwarecodes als Zerlegekrierium angewendet werden.

Alternativ oder ergänzend können Zerlegekriterien durch formale Kriterien gebildet werden, also Kriterien, die formelhaft auf beliebige Teile einer zu testenden Implementierung angewendet werden, z. B. Anhand von Dateiablage, syntaktischen Elementen, Namen usw. Beispielsweise kann jede Funktion eines C-Programms zu einem zu testenden Abschnitt werden.

Alternativ oder ergänzend können Zerlegekriterien frei vorgebbar sein. Dabei erfolgt die Zerlegung anhand manuell definierter Grenzen, z. B. einer konkreten Liste von gewünschten Einsprungpunkten und/oder konkret benannten Teilen, die nicht abgetrennt werden sollen.

Die Zerlegekriterien können durch formale Kriterien gebildet und frei vorgebbar sein, um auch komplexe Implementierungen effizient testen zu können.

Es können ein oder auch mehrere Zerlegekriterien zur Anwendung kommen. In einer Konfigurationsphase kann definiert werden, welche der Zerlegekriterien und/oder in welcher Form (z.B. Schwellenwerte, Grenzwerte) diese zur Anwendung kommen sollen.

Vorzugsweise erfolgt das Erstellen jeweils einer Testumgebung für jeweils einen Softwarecodeabschnitt aller Softwarecodeabschnitte iterativ, indem eine initial automatisch erstellte Testumgebung sukzessive automatisch durch fehlende Elemente ergänzt wird.

Die initiale Testumgebung kann leer sein oder aus einer wie auch immer gearteten Analyse entstanden sein. Alternativ kann mit einer manuell festgelegten Testumgebung begonnen werden. Vorteilhafterweise werden die vorstehend genannten Festlegungen in Abhängigkeit von Sprache und Implementierung bestimmt. Beispielsweise kann in C/C++ mit einer leeren Testumgebung begonnen werden. Scheitert das Ausführen der Testumgebung beispielsweise aufgrund einer fehlenden Deklaration einer Funktion, wird eine solche Funktion automatisch hinzugefügt und die Lauffähigkeit erneut geprüft. Das Verhalten der entsprechenden Funktion (z. B. Rückgabewerte) kann von zusätzlichen, testabhängigen Eingangsdaten gesteuert werden, die dann im folgenden Schritt ebenfalls automatisch erzeugt werden. Entsprechend können fehlende Typdefinitionen, fehlende globale Variablen usw. verarbeitet werden. Elemente, deren Definitionen nicht offensichtlich sind (zum Beispiel Datentypen), können entsprechend der Definition der ursprünglichen Software in der Testumgebung angelegt werden. Dies ist mittels syntaktischer Analyse der bisherigen Software möglich.

Fehlende Elemente sind z. B. identifizierbar durch Diagnosemeldungen beim Versuch der Ausführung. Auch dies ist sprachabhängig. In C/C++ können beispielsweise die Fehlermeldungen des Compilers und/oder Linkers durch das erfindungsgemäße Verfahren prozessiert werden. Das Ergänzen kann in C/C++ erfolgen, indem z. B entsprechende Objekte oder Typen mit den entsprechenden Datentypen bzw. Methoden und Signaturen der Testumgebung hinzugefügt werden. Die notwendigen Informationen können aus Fehlermeldungen oder dem Gesamtcode der Implementierung extrahiert werden.

Da auch bei beliebiger Zerlegung der Software jeder Softwareteil nur begrenzte Abhängigkeiten aufweisen kann, konvergiert eine iterative Erstellung der Testumgebung nach endlich vielen Schritten.

Das Erstellen von Teststimuli bzw. Eingangsdaten kann vorzugsweise durch einen Algorithmus zur sogenannten "symbolischen Ausführung" (symbolic execution) auf jeweils einem zerlegten Softwarecodeabschnitt inklusive der erstellten Testumgebung ausgeführt werden. Für weitere Details zur symbolischen Ausführung verweisen wir auf KLEE: *"*Unassisted and Automatic Generation of High-Coverage Tests for Complex Systems Programs", Cristian Cadar, Daniel Dunbar, Dawson Engler, 8th USENIX Symposium on Operating Systems Design and Implementation, 2008*.* Bei der symbolischen Ausführung werden anstelle von konkreten Eingabewerten für die zu testende Einheit (Softwarecodeabschnitt) abstrakte Eingangsdaten, z.B. ein logischer Ausdruck, insbesondere in Form eines Terms und/oder einer Funktion verwendet und der jeweilige Softwarecodeabschnitt (z.B. die Unit) wird einer Pfadanalyse unterzogen. Als "Pfad" wird in diesem Zusammenhang ein Ausführungspad des Programms zur Laufzeit verstanden, also welche Programmverzweigungen aufgrund der Eingangsdaten verfolgt werden. Wird ein Programm entlang eines Pfades symbolisch ausgeführt, so kann zu jedem Punkt in dieser Ausführung einen Wert und eine Pfadbedingung assoziiert gespeichert werden.

Das Ausführen eines Testverfahrens auf dem Produktivsystem und/oder auf dem geänderten System inklusive der erstellten Testumgebung kann für jeden zerlegten Softwarecodeabschnitt und/oder für jeden Satz von dazu erstellten Teststimuli bzw. Eingangsdaten, insbesondere separat und/oder einzeln, erfolgen. Da diese Kombinationen unterschiedliche Aspekte testen, müssen sie separat ausgeführt werden. Alternativ oder ergänzend kann das Ausführen des Testverfahrens auf dem geänderten System inklusive der erstellten Testumgebung für jeden zerlegten Softwarecodeabschnitt und/oder für jeden Satz von dazu erstellten Teststimuli bzw. Eingangsdaten, insbesondere separat und/oder einzeln, erfolgen.

Eine Parallelisierung (der Ausführung der oben genannten unterschiedlichen Kombinationen der Testverfahren) ist möglich und sinnvoll; die Unabhängigkeit der Ausführungen muss aber auch dabei sichergestellt sein. Testergebnisse und -Ausführungen müssen grundsätzlich unabhängig von zuvor und/oder parallel ausgeführten Tests sein, um eine wechselseitige Beeinflussung auszuschließen.

Alternativ oder kumulativ kann das erzeugte erste und/oder das erzeugte zweite Testergebnis als digitaler eindeutiger Code, insbesondere als Prüfsumme, digital verarbeitet und/oder gespeichert werden. Dies hat den Vorteil, dass weniger Speicherplatz erforderlich ist und, dass ggf. die Performanz des Verfahrens gesteigert werden kann, da der Abgleich von Prüfsummen schneller ausführbar sein kann, als von umfangreicheren Testergebnissen. Die Verwendung (Verarbeitung und/oder Speicherung) des digitalen eindeutigen Codes kann abhängig von dem zu testenden System bestimmt werden. Es ist auch möglich, das erzeugte erste und/oder zweite Testergebnis zusätzlich zu dem jeweiligen digitalen eindeutigen Code zu speichern, beispielsweise zur Analyse bei Abweichungen.

Das Ausgeben eines Testergebnisses kann die Ausgabe einer Testabdeckung umfassen. Damit kann die Zuverlässigkeit des Verfahrens verbessert werden.

Das Erstellen jeweils einer Testumgebung für jeweils einen Softwarecodeabschnitt kann eine Benutzerinteraktion umfassen. Insbesondere kann das Erstellen der Testumgebung ein vollständiges oder teilweises Übernehmen von Softwarecodeabschnitten des Produktivsystems (der unveränderten Implementierung) in die Testumgebung umfassen. Insbesondere wenn ein durch die Testdatenerstellung (Stimuli-Generierung) gesteuertes Verhalten der Testumgebung nicht zu einer adäquaten Testabdeckung führt (z. B. weil die Verfahren zur Stimuli-Generierung ansonsten eine zu hohe Rechenzeit / Speicherplatz benötigen), kann es sinnvoll sein, die Testumgebung zumindest teilweise aus Teilen (z. B. einzelnen Funktionen) der ursprünglichen Implementierung (z.B. Legacy Code) zusammenzusetzen.

Es ist auch möglich, einzelne, automatisierte Schritte des vorstehend beschriebenen Verfahrens zur Funktionalitätsprüfung durch manuelle Benutzerinterkationen zu ersetzen, z. B. beim Erstellen der Teststimuli bzw. Eingangsdaten bzw. Stimuli oder der Testumgebung oder durch ein manuelles Anpassen der automatisiert gewonnenen Artefakte, z. B. Ergänzung der Stimuli, um die Testabdeckung zu erhöhen.

Alternativ oder kumulativ können automatisch weitere Maßnahmen anhand der gefundenen Abweichungen bei dem Vergleich zwischen dem ersten und dem zweiten Testergebnis veranlasst werden, wie beispielsweise das Erstellen manueller Tests, ein zusätzliches Review kritischer Details und/oder eine Anpassung der geänderten Implementierung.

Alternativ oder kumulativ können automatisch weitere Maßnahmen in Antwort auf die erzielte Testabdeckung angestoßen werden, wie beispielsweise das Ausführen von zusätzlichen manuellen Tests bei geringer Testabdeckung und/oder einer Refaktorisierung der Software anhand der erzielten Testabdeckung. "Refaktorisierung" (refactoring) des Softwarecodes meint in diesem Kontext ein Überarbeiten der Struktur des Quellcodes ohne, dass an dessen Funktionalität oder beobachtbarem Verhalten etwas geändert wird. Ziel der Refaktorisierung ist es, den Code besser zu strukturieren, lesbarer zu gestalten, insbesondere auch um ihn leichter wartbar zu machen und/oder besser weiterentwickeln zu können und die Software performanter zu machen.

Alternativ oder kumulativ können die hier vorgeschlagene automatisch generierte Softwarecode-Zerlegung in Softwarecodeabschnitte und erstellte Testumgebungen als Grundlage für Refaktorisierungen verwendet werden. Zusätzlich zu Abhängigkeiten zwischen den Softwareteilen ist der Grad an Gemeinsamkeiten zwischen den jeweiligen Testumgebungen ein hilfreiches Maß für Refaktorisierungen.

Mit der erfindungsgemäßen Lösung wird es möglich, dass die Felddaten oder die Gesamtheit der Felddaten in Form von Felderfahrung zumindest indirekt in den Test miteinfließt. Mit "indirekt einfließen" ist in diesem Kontext gemeint, dass die Felddaten nicht zwingend explizit als "Argument" im Test benötigt werden. Die Felddaten fließen nur implizit bei dem Test ein, insbesondere als Datensatz, der repräsentiert, dass bei einer bestimmten Menge von Maschinen in einem bestimmten Zeitraum keine Fehler beobachtet werden konnten bzw. das System fehlerfrei lief. Alternativ können die Felddaten in einer Weiterbildung der Erfindung aber auch direkt und/oder explizit bei dem Test prozessiert werden. Dazu können zu bereits identifizierten Softwareteilen Stimuli und optional Ergebnisse im Rahmen der Ausführung im Feld abgespeichert werden. Diese können zur Ermittlung von Testfällen und/oder von Ergebnissen auf dem Produktivsystem bzw. zum Vergleich mit Ergebnissen des veränderten Systems herangezogen werden.

Damit wird es auch möglich, bisherige manuelle Systemtests durch automatisch generierte Softwaretests zu ersetzen. Damit können erstellte, automatisiert wiederholbare Softwaretests auch ohne Änderung der Implementierung die Notwendigkeit manueller Tests reduzieren.

In einem weiteren Aspekt betrifft die Erfindung ein Computerprogramm, wobei das Computerprogramm in eine Speichereinheit eines Computers ladbar ist und Programmcodeabschnitte enthält, um den Computer zu veranlassen, das Verfahren zur Funktionalitätsprüfung, wie vorstehend beschrieben, auszuführen, wenn das Computerprogramm in dem Computer ausgeführt wird.

In einem weiteren Aspekt betrifft die Erfindung ein Testsystem, das auch als Testgerät ausgebildet sein kann, und zur Ausführung des vorstehend beschriebenen Verfahrens bestimmt ist. Das Testsystem dient zur Funktionalitätsprüfung von Softwarecode eines Produktivsystems für ein medizintechnisches Gerät. Das Testsystem wird bevorzugt dann angewendet, wenn aus dem Produktivsystem durch Änderungen an dessen Hardware und/oder Software ein geändertes System erzeugt wurde. Das Produktivsystem kann mit Hardware und Softwarecode betrieben und/oder gesteuert werden. Das geänderte System kann mit geänderter Hardware und/oder mit geändertem Softwarecode betrieben und/oder ausgeführt werden. Das Testsystem ist ausgebildet mit:
- Einer ersten Testeinheit, die ein Ausführen eines Testverfahrens auf dem Produktivsystem veranlasst und, die zum Erzeugen eines ersten Testergebnisses bestimmt ist;
- Einer zweiten Testeinheit, die ein Ausführen des Testverfahrens auf dem geänderten System veranlasst und, die zum Erzeugen eines zweiten Testergebnisses bestimmt ist;
- Einer Recheneinheit, die zum Vergleichen des ersten Testergebnisses mit dem zweiten Testergebnis bestimmt ist, um eine spezifikationskonforme Funktionalität des geänderten Systems zu prüfen;
- Einer Ausgabeschnittstelle zum Ausgeben eines Testergebnisses.

Für das Testsystem bestehen unterschiedliche Implementierungsvarianten. So kann das Testsystem in Form eines Testgeräts ausgebildet sein. Alternativ oder kumulativ kann das Testsystem als separates Zusatzgerät bereitgestellt werden, das bei Bedarf (also zeitweise zum Testen) über geeignete logische und/oder physikalische Schnittstellen an das Produktivgerät angeschlossen werden kann. Alternativ oder kumulativ kann das Testsystem direkt auf dem Produktivgerät selbst, also z.B. dem Dialysegerät installiert sein.

Die erste Testeinheit kann direkt auf dem Produktivsystem installiert sein und das Ausführen des Testverfahrens veranlassen und/oder initiieren und/oder steuern. Die erste Testeinheit kann alternativ oder kumulativ auf einem separaten Gerät installiert sein (separat vom Produktivsystem) und nur in Datenverbindung mit dem Produktivsystem stehen.

Die zweite Testeinheit kann direkt auf dem geänderten System (geänderte Hardware und/oder geänderte Software, z.B. neues Software-Release oder Patch) installiert sein und das Ausführen des Testverfahrens veranlassen und/oder initiieren und/oder steuern. Die zweite Testeinheit kann alternativ oder kumulativ auf einem separaten Gerät installiert sein (separat vom geänderten System) und nur in Datenverbindung mit dem geänderten System stehen.

Die Recheneinheit kann auf der Hardware "deployed" sein, auf der auch die erste und/oder zweite Testeinheit installiert ist oder kann auf einer separaten Hardware installiert sein mit entsprechender Datenanbindung.

Die Ausgabeschnittstelle kann auf dem Produktivsystem und/oder dem geänderten System und/oder einem weiteren Gerät ausgebildet sein, wie z.B. einem mobilen Endgerät, wie z.B. einem Mobilfunktelefon oder einem Laptop oder dergleichen. Das Testsystem kann als verteiltes System ausgebildet sein.

In einem weiteren Aspekt betrifft die Erfindung die Verwendung des oben beschrieben Verfahrens und/oder Testsystem zur Steuerung weiterer Maßnahmen. Weitere Maßnahmen können z. B. eine Refaktorisierung und/oder eine manuelle Testerstellung betreffen.

In einem weiteren Aspekt betrifft die Erfindung ein medizinisches Gerät mit einem Testsystem oder mit dessen Einheiten /Teilen, wie vorstehend beschrieben.

In einem weiteren Aspekt betrifft die Erfindung ein medizinisches Behandlungssystem zur medizinischen Behandlung von Patienten, mit einer elektronischen Einheit, wobei die Funktionalitätsprüfung von Funktionen der elektronischen Einheit durch ein Verfahren zur Funktionalitätsprüfung von Softwarecode, wie vorstehend beschrieben, ausgeführt wird.

In der folgenden detaillierten Figurenbeschreibung werden nicht einschränkend zu verstehende Ausführungsbeispiele mit deren Merkmalen und weiteren Vorteilen anhand der Zeichnung besprochen.

### Kurze Beschreibung der Figuren

- Fig. 1a: zeigt ein Produktivsystem P, aus dem durch zumindest eine Änderung an einem Softwarecode, der auf einer Hardware installiert ist, ein geändertes System P' erzeugt wird sowie ein Testverfahren zur Ausführung auf dem Produktivsystem P und dem geänderten System P' zur Erzeugung von einem ersten und zweiten Testergebnis;
- Fig. 1b: zeigt ein Produktivsystem P, aus dem durch zumindest eine Änderung an der Hardware, auf der ein Softwarecode installiert ist, ein geändertes System P' erzeugt wird sowie ein Testverfahren zur Ausführung auf dem Produktivsystem P und dem geänderten System P' zur Erzeugung von einem ersten und zweiten Testergebnis;
- Fig. 2: ist ein Ablaufdiagramm eines Verfahrens 200 gemäß einer bevorzugten Ausführungsform der Erfindung;
- Fig. 3a: zeigt ein Ausführungsbeispiel, bei dem das Produktivsystem P auf einem medizinischen Gerät DG installiert ist und
- Fig. 3b: ein weiteres Ausführungsbeispiel, bei dem das Produktivsystem P auf einer separaten Recheneinheit für ein medizinisches Gerät DG betrieben wird;
- Fig. 4: ist eine schematische Übersichtsdarstellung eines Testsystems 400 mit weiteren Bestandteilen;
- Fig. 5: ist eine Ausführungsform, bei der das Testsystem 400 auf dem medizinischem Gerät DG implementiert ist und
- Fig. 6: ist eine weitere Ausführungsform, bei der das Testsystem 400 als verteiltes System ausgebildet ist;
- Fig. 7: zeigt ein Ablaufdiagramm eines Verfahrens 700 gemäß einer bevorzugten Ausführungsform der Erfindung und
- Fig. 8: zeigt eine schematische Übersichtsdarstellung eines Testsystems 400 von Fig. 4 in einer weiteren Ausführungsform.

### Detaillierte Beschreibung der Erfindung

Die vorliegende Erfindung betrifft einen computer-implementiertes Verfahren zur Funktionsverifikation bzw. -überprüfung von sogenanntem Legacy Softwareprogrammcode auf z.B. modernisierten Hardware-Implementierungen unter Verwendung von Felddaten.

Medizintechnische Geräte DG, wie insbesondere Dialysegeräte oder Blutbehandlungsgeräte, umfassen eine Vielzahl von mechanischen, elektronischen und/oder physikalischen Komponenten. Die elektronischen Komponenten können in Form einer elektronischen Einheit zusammengefasst werden. Die elektronische Einheit kann zum Betreiben und/oder Steuern eines Produktivsystems P verwendet werden. Die elektronische Einheit und/oder das Produktivsystem P kann eine Hardware HW umfassen, auf der ein Softwarecode SW installiert ist. Im Laufe der Lebenszeit des medizintechnischen Gerätes DG und/oder des Produktivsystems P für das medizintechnische Gerät DG ist das Produktivsystem P Änderungen unterworfen. Zum einen können Änderungen an dem Softwarecode SW und/oder zum anderen an der Hardware HW vorgenommen werden; beides führt jeweils zu einem geänderten System P'.

Da das Produktivsystem P grundsätzlich für ein medizinisches Gerät DG verwendet wird, müssen jegliche Änderungen am Produktivsystem P überprüft werden, insbesondere ob sie den regulatorischen Anforderungen in der Medizintechnik genügen.

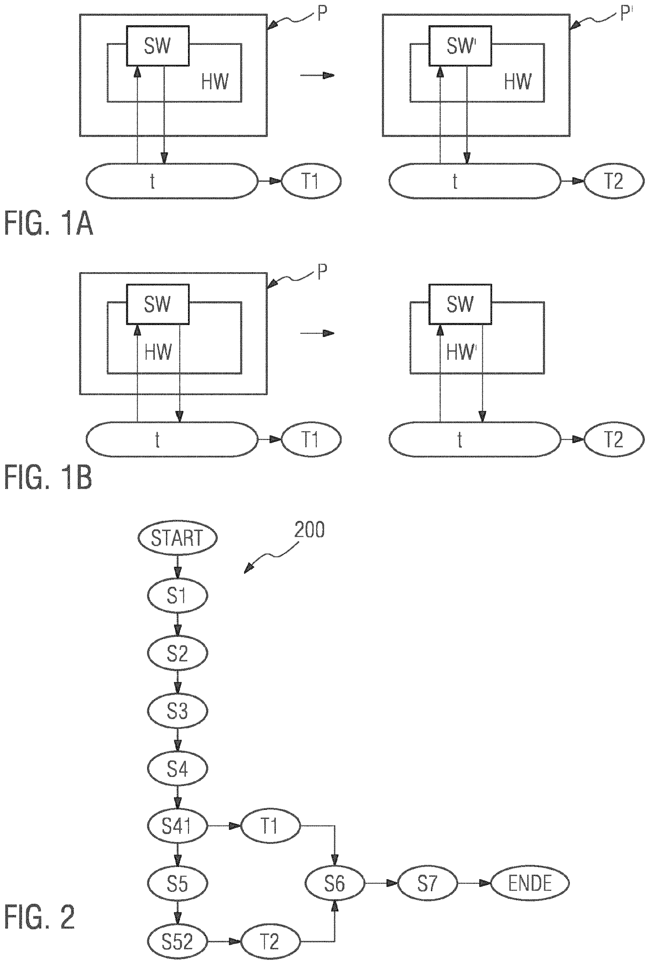

**Figur 1A** zeigt nun ein Ausführungsbeispiel, bei dem die Änderungen an dem Softwarecode SW -> SW' vorgenommen werden, während die Hardware HW unverändert verbleibt. Dies kann beispielsweise durch das Aufspielen eines Patches oder eines neuen Softwarereleases veranlasst sein.

**Figur 1B** zeigt ein weiteres Ausführungsbeispiel, bei dem die Änderungen nicht oder nicht nur an einem Softwarecode SW vorgenommen werden, sondern bei dem eine neue Hardware HW' verwendet wird.

In beiden Fällen wird aus dem ursprünglichen Produktivsystem P ein geändertes System P' erzeugt. Das Produktivsystem P und das geänderte System P' können aber zeitgleich existieren. Es gilt jeweils, das geänderte Produktivsystem P' zu testen.

Dazu wird der Softwarecode einem Testverfahren t unterzogen.

Im Ausführungsbeispiel von Figur 1A wird sowohl der Softwarecode SW auf dem alten unveränderten Produktivsystem P mit dem Testverfahren t getestet (dies ist in Figur 1A auf der linken Seite dargestellt) als auch der geänderte Softwarecode SW' des geänderten Produktivsystems P' (dies ist in Figur 1A auf der rechten Seite dargestellt). Das auf dem Produktivsystem P ausgeführte Testverfahren t erzeugt ein erstes Testergebnis T1 und das auf dem geänderten System P' ausgeführte Testverfahren t erzeugt ein zweites Testergebnis T2.

Im Ausführungsbeispiel von Figur 1B wird sowohl der Softwarecode SW auf dem alten unveränderten Produktivsystem P mit dem Testverfahren t getestet (dies ist in Figur 1A auf der linken Seite dargestellt) als auch der Softwarecode SW auf der geänderten Hardware HW' des Produktivsystems P' (dies ist in Figur 1A auf der rechten Seite dargestellt). Das auf dem Produktivsystem P ausgeführte Testverfahren t erzeugt ein erstes Testergebnis T1 und das auf dem geänderten System P' ausgeführte Testverfahren t erzeugt ein zweites Testergebnis T2.

In einem späteren Verfahrensschritt des Verfahrens zur Funktionalitätsprüfung kann das erste Testergebnis T1 mit dem zweiten Testergebnis T2 auf Übereinstimmung verglichen werden, um eine (finales) Testergebnis TE zu erzeugen und/oder auszugeben.

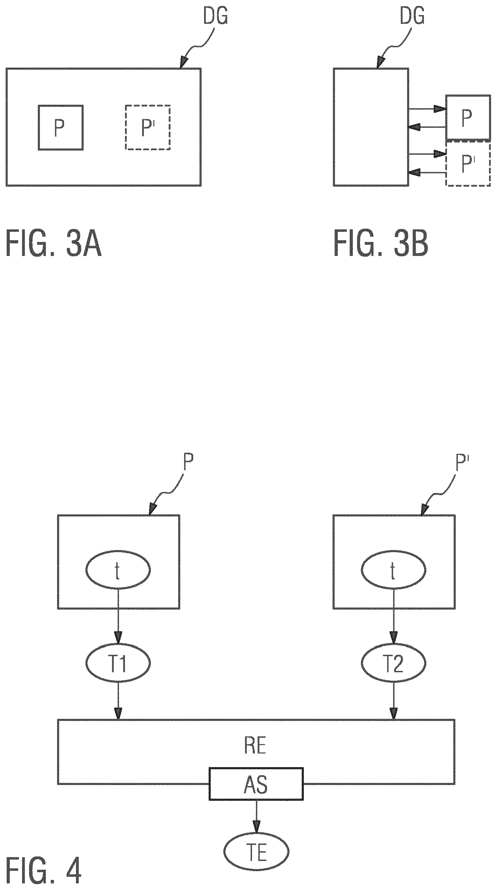

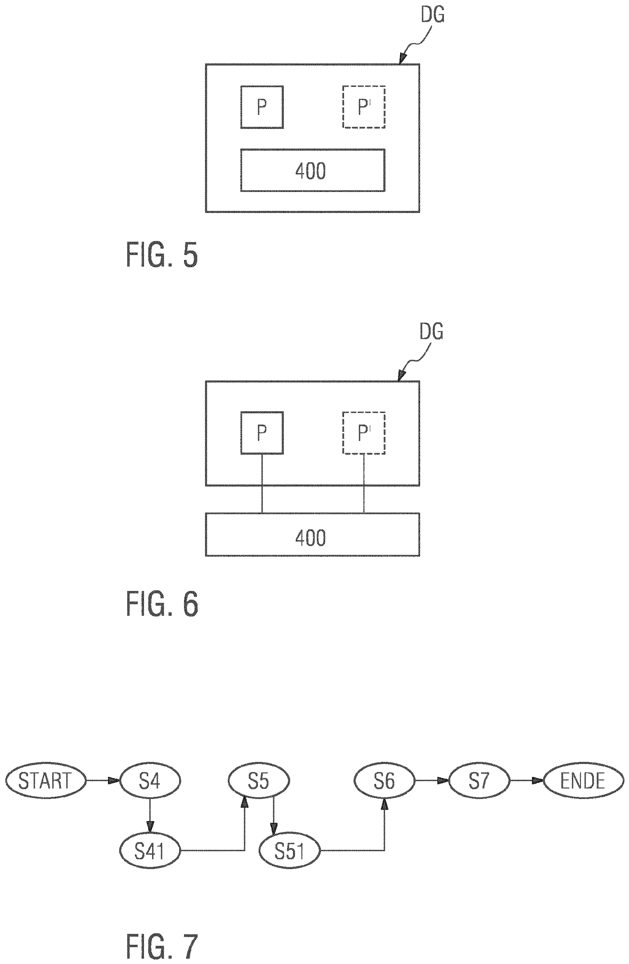

**Figur 7** beschreibt ein Ablaufdiagramm eines Verfahrens 700 zur Funktionalitätsprüfung gemäß einem Ausführungsbeispiel. Nach dem Start des Verfahrens kann in Schritt S4 das Ausführen des Testverfahrens t auf dem Produktivsystem P ausgeführt werden, um in Schritt S41 das erste Testergebnis T1 zu erzeugen. Anschließend kann in Schritt S5 das Testverfahren t auf dem geänderten System P' ausgeführt werden, um in Schritt S51 das zweite Testergebnis T2 zu erzeugen. In Schritt S6 kann das erste Testergebnis T1 mit dem zweiten Testergebnis T2 verglichen werden. Der Vergleich kann vorzugsweise durch einen Algorithmus ausgeführt werden. In Schritt S7 kann das finale Testergebnis TE ausgegeben werden, beispielsweise auf einer Ausgabeschnittstelle AS (in Figur 4 gezeigt).

**Figur 2** zeigt ein Ablaufdiagramm eines Verfahrens 200 zur Funktionalitätsprüfung gemäß einem weiteren Ausführungsbeispiel.

Nach dem Start des Verfahrens kann in Schritt S1 ein Zerlegen des Softwarecodes SW in Softwarecodeabschnitte erfolgen. Dies wird vorzugsweise algorithmisch und automatisch ausgeführt. Das automatische Zerlegen des Softwarecodes SW kann beispielsweise anhand von formalen, vorkonfigurierbaren und vom Anwender auswählbaren Zerlegekriterien erfolgen, die ihm oder ihr auf einer Benutzerschnittstelle zur Auswahl angeboten werden, nachdem sie in einer Konfigurationsphase konfiguriert worden sind. Diese Zerlegekriterien können beispielsweise eine Dateizugehörigkeit, eine Namensgebung und/oder syntaktische und/oder semantische Eigenschaften des Softwarecodes SW und/oder der Softwarecodeabschnitte betreffen. Beispielsweise kann es sich um Funktionsgrenzen, Schleifenkörper, Blockgrenzen handeln. Kumulativ oder alternativ kann das Zerlegen nach Zeilenanzahl des Softwarecodes SW ausgeführt werden.

In einem nachfolgenden Verfahrensschritt S2 kann automatisch eine Testumgebung für jeweils einen der zerlegten Softwarecodeabschnitte aus der Menge aller Softwarecodeabschnitte erstellt werden. Das automatische Erstellen der Testumgebung (auch als sogenannter "mock" bezeichnet) kann interaktiv ausgeführt werden, indem die Lauffähigkeit des jeweiligen Softwarecodes mit der bisherigen (unveränderten) Testumgebung geprüft wird und die Testumgebung gegebenenfalls um fehlende Elemente ergänzt wird.

In einem weiteren nachfolgenden Verfahrensschritt S3 können Teststimuli bzw. Eingangsdaten automatisch erstellt werden, die zur Ausführung des Testverfahrens t verwendet werden. Für jeden identifizierten bzw. zerlegten Softwarecodeabschnitt inklusive Testumgebung können nun durch sogenannte "symbolische Ausführung" ein oder mehrere Sätze von Stimuli bzw. Eingangsdaten/Teststimuli bzw. Eingangsdaten automatisiert erzeugt werden. Damit werden Teststimuli bzw. Eingangsdaten für den Softwarecodeabschnitt und gegebenenfalls für die Testumgebung erzeugt.

In dem nachfolgenden Verfahrensschritt S4 wird das Testverfahren t auf das Produktivsystem mit den im vorhergehenden Verfahrensschritt erzeugten Teststimuli angewendet, um ein erstes Testergebnis T1 zu erzeugen.

Alternativ kann das Testverfahren t auf dem Produktivsystem mit den Felddaten angewendet werden. In der vorstehend genannten Alternative, entfällt aber das Erzeugen der Stimuli mittels symbolischer Ausführung/Simulation.

Da bekannt ist, dass das Produktivsystem P in der unveränderten Version fehlerfrei läuft, repräsentiert das erste Testergebnis T1 per definitionem einen fehlerfreien Zustand des Produktivsystems P. Dieser fehlerfreie Zustand kann als Referenz bzw. Benchmark und zum Vergleich mit dem zweiten Testergebnis T2 verwendet werden. Das zweite Testergebnis T2 wird automatisch erzeugt durch Ausführen desselben Testverfahrens t auf dem geänderten System P'.

Im Schritt S6 kann dann das erste Testergebnis T1 mit dem zweiten Testergebnis T2 auf Übereinstimmung verglichen werden, um in Schritt S7 ein finales Testergebnis TE auszugeben, was somit implizit auf dem Zustand basiert, der in den erfassten Felddaten, repräsentiert ist (nämlich dem bisherigen - ggf. fehlerfreien - Betrieb des Produktivsystems).

In **Figur 3A** ist eine Implementierungsmöglichkeit gezeigt, bei der das Produktivsystem P und/oder das geänderte System P' direkt und unmittelbar auf dem medizinischen Gerät DG implementiert ist. Da das geänderte System P' durch Änderungen an dem Softwarecode SW des Produktivsystems und/oder an der Hardware HW des Produktivsystems hervorgeht, ist das geänderte System gestrichelt dargestellt.

**Figur 3B** hingegen zeigt eine alternative Implementierungsmöglichkeit, bei der das Produktivsystem P und/oder das geänderte System P' nicht direkt auf dem medizinischen Gerät DG implementiert ist, sondern für das medizinische Gerät DG betrieben wird/werden. Das Produktivsystem P und/oder das geänderte System P' können auf einem separaten Computer bzw. auf einer separaten Computereinheit (z.B. speicherprogrammierbare Steuerung, SPL) implementiert sein, die in Datenverbindung mit dem medizinischen Gerät DG steht. Wie in Fig. 3A ist das geänderte System wieder gestrichelt dargestellt.

**Figur 4** zeigt ein strukturelles Blockschaltbild eines Testsystems 400, bei dem das Produktivsystem P auf der linken Seite und das geänderte System P' auf der rechten Seite dargestellt ist. Nach Ausführung des Testverfahrens t auf dem Produktivsystem P wird das erste Testergebnis T1 automatisch erzeugt und nach Ausführung desselben Testverfahrens t auf dem geänderten System P' wird automatisch das zweite Testergebnis T2 erzeugt. Daraufhin kann auf einer Recheneinheit RE ein Vergleichen des ersten Testergebnisses T1 mit dem zweiten Testergebnis T2 erfolgen, um das (finale) Testergebnis TE über die Ausgabeschnittstelle AS auszugeben. Das Testergebnis TE ist deshalb ein finales Testergebnis.

**Figur 5** zeigt ein Implementierungsbeispiel, bei dem das Produktivsystem P und/oder das geänderte System P' auf dem medizinischen Gerät DG implementiert ist/sind und bei dem das Testsystem 400 ebenfalls auf dem medizinischen Gerät DG implementiert ist.

Im Unterschied dazu zeigt das Implementierungsbeispiel von **Figur 6****,** dass das Produktivsystem P und/oder das geänderte System P' auf dem medizinischen Gerät DG implementiert sein können, während das Testsystem 400 als verteiltes System auf einem separaten Computer implementiert ist, der in Datenverbindung mit dem Produktivsystem P, P' des medizinischen Gerätes DG steht.

Abschließend sei darauf hingewiesen, dass die Beschreibung der Erfindung und die Ausführungsbeispiele grundsätzlich nicht einschränkend in Hinblick auf eine bestimmte physikalische Realisierung der Erfindung zu verstehen sind. Alle in Verbindung mit einzelnen Ausführungsformen der Erfindung erläuterten und gezeigten Merkmale können in unterschiedlicher Kombination in dem erfindungsgemäßen Gegenstand vorgesehen sein, um gleichzeitig deren vorteilhafte Wirkungen zu realisieren.

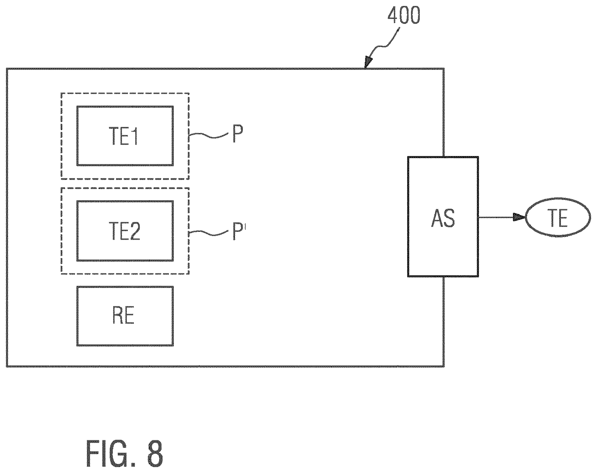

**Figur 8** zeigt ein Testsystem 400 mit einer ersten Testeinheit TE1, einer zweiten Testeinheit TE2 und einer Recheneinheit RE und einer Ausgabeschnittstelle AS zur Ausgabe eines Testergebnisses TE. Die erste Testeinheit TE1 kann auf dem Produktivsystem P implementiert sein (deshalb in Fig. 8 gestrichelt dargestellt, da dies nur optional ist) und die zweite Testeinheit TE2 kann auf dem geänderten System P' implementiert sein (deshalb in Fig. 8 ebenfalls gestrichelt dargestellt, da dies nur optional ist), um das Testverfahren auf dem Produktivsystem P bzw. auf dem geänderten System P' anzustoßen. Die Ausführungen der Testverfahren laufen somit auf unterschiedlichen Systemen. Die Ausführungen der Testverfahren können zeitgleich, parallel oder sequentiell ausgeführt werden.

Der Schutzbereich der vorliegenden Erfindung ist durch die Ansprüche gegeben und wird durch die in der Beschreibung erläuterten oder den Figuren gezeigten Merkmale nicht beschränkt.

Für einen Fachmann ist es insbesondere offensichtlich, dass die Erfindung nicht nur für Dialysegeräte angewendet werden kann, sondern auch für andere medizintechnische Geräte (wie beispielsweise Blutbehandlungsgeräte oder Geräte zur Behandlung von Nierenerkrankungen) oder für nicht-medizintechnische Geräte, die eine elektronische Einheit umfassen und/oder von einem Produktivsystem betrieben und/oder gesteuert werden. Des Weiteren können die Bauteile des Testsystems 400 auf mehreren physikalischen Produkten verteilt realisiert sein.

### BEZUGSZEICHENLISTE

- DG: medizintechnisches Gerät, insbesondere Dialysegerät
- P: Produktivsystem
- P': geändertes System
- SW: Softwarecode bzw. Software
- HW: Hardware
- SW': geänderter Softwarecode
- HW': geänderte Hardware
- RE: Recheneinheit
- AS: Ausgabeschnittstelle
- t: Testverfahren
- T1: erstes Testergebnis
- T2: zweites Testergebnis
- TE: (finales) Testergebnis

- S1: Zerlegen
- S2: Erstellen einer Testumgebung
- S3: Erstellen von Testdaten
- S4: Ausführen des Testverfahrens auf dem Produktivsystem P
- S41: Erzeugen eines ersten Testergebnisses für das in Schritt S4 ausgeführte Testverfahren
- S5: Ausführen des Testverfahrens auf dem geänderten System P'
- S51: Erzeugen eines zweiten Testergebnisses für das in Schritt S5 ausgeführte Testverfahren

- 400: Testsystem
- TE1: erste Testeinheit
- TE2: zweite Testeinheit
- 200: Verfahren zur Funktionalitätsprüfung von Softwarecode gemäß einer ersten Ausführungsform der Erfindung
- 700: Verfahren zur Funktionalitätsprüfung von Softwarecode gemäß einer zweiten Ausführungsform der Erfindung

## Patentansprüche

1. Computer-implementiertes Verfahren zur Funktionalitätsprüfung von Softwarecode (SW) eines Produktivsystems (P) für ein medizintechnisches Gerät (DG), wenn aus dem Produktivsystem (P) durch Änderungen an dessen Hardware (HW) und/oder an dessen Softwarecode (SW) ein geändertes System (P') erzeugt wurde, mit den folgenden Verfahrensschritten:
- Ausführen (S4) eines Testverfahrens (t) auf dem Produktivsystem (P) und Erzeugen (S41) eines ersten Testergebnisses (T1);
- Ausführen (S5) des Testverfahrens (t) auf dem geänderten System (P') und Erzeugen (S51) eines zweiten Testergebnisses (T2);
- Vergleichen (S6) des ersten Testergebnisses (T1) mit dem zweiten Testergebnis (T2), um eine spezifikationskonforme Funktionalität des geänderten Systems (P') zu prüfen;
- Ausgeben (S7) eines Testergebnisses (TE).

2. Verfahren nach Anspruch 1, bei dem das Testverfahren (t) ein Unittest ist.

3. Verfahren nach einem der vorangehenden Ansprüche, bei dem das Verfahren zumindest einen der folgenden Schritte umfasst, die alle automatisch ausgeführt werden:
- Zerlegen (S1) des Softwarecodes (SW) in Softwarecodeabschnitte;
- Erstellen (S2) jeweils einer Testumgebung für jeweils einen Softwarecodeabschnitt aller Softwarecodeabschnitte und/oder
- Erstellen (S3) von Teststimuli zur Ausführung des Testverfahrens (t).

4. Verfahren nach einem der vorangehenden Ansprüche, bei dem ein Zerlegen (S1) des Softwarecodes (SW) in Softwarecodeabschnitt automatisch erfolgt, durch Anwendung von formalen und/oder vom Anwender konfigurierbaren Zerlegekriterien.

5. Verfahren nach dem unmittelbar vorangehenden Anspruch, wobei die Zerlegekriterien eine Dateizugehörigkeit, eine Namensgebung und/oder syntaktische und/oder semantische Eigenschaften des Softwarecodes (SW) und/oder der Softwarecodeabschnitte auswerten.

6. Verfahren nach einem der vorangehenden Ansprüche, bei dem ein Erstellen (S2) jeweils einer Testumgebung für jeweils einen Softwarecodeabschnitt aller Softwarecodeabschnitte iterativ erfolgt, indem eine initial automatisch erstellte Testumgebung sukzessive automatisch durch fehlende Elemente ergänzt wird.

7. Verfahren nach einem der vorangehenden Ansprüche 3 bis 6, bei dem das Erstellen von Teststimuli (S3) durch einen Algorithmus zur symbolischen Ausführung auf jeweils einen zerlegten Softwarecodeabschnitt inklusive der erstellten Testumgebung ausgeführt wird.

8. Verfahren nach einem der vorangehenden Ansprüche 3 bis 7, bei dem das Ausführen (S4) des Testverfahrens (t) auf dem Produktivsystem (P) und/oder auf dem geänderten System (P') inklusive der erstellten Testumgebung für jeden zerlegten Softwarecodeabschnitt und/oder für jeden Satz von dazu erstellten Teststimuli erfolgt.

9. Verfahren nach einem der vorangehenden Ansprüche, bei dem das erzeugte erste Testergebnis (T1) und das erzeugte zweite Testergebnis (T2) als digitaler eindeutiger Code, insbesondere als Prüfsumme, verarbeitet und/oder gespeichert wird.

10. Verfahren nach einem der vorangehenden Ansprüche, bei dem das Ausgeben (S7) eines Testergebnisses (TE) die Ausgabe einer Testabdeckung umfasst.

11. Verfahren nach einem der Ansprüche 3 bis 10, bei dem das Erstellen (S2) jeweils einer Testumgebung für jeweils einen Softwarecodeabschnitt ein vollständiges oder teilweises Übernehmen von Softwarecodeabschnitten des Produktivsystems (P) in die Testumgebung umfasst.

12. Computerprogramm, wobei das Computerprogramm in eine Speichereinheit eines Computers ladbar ist und Programmcodeabschnitte enthält, um den Computer zu veranlassen, das Verfahren zur Funktionalitätsprüfung gemäß einem der vorangehenden Verfahrensansprüche auszuführen, wenn das Computerprogramm in dem Computer ausgeführt wird.

13. Testsystem (400) zur Funktionalitätsprüfung von Softwarecode (SW) eines Produktivsystems (P) für ein medizintechnisches Gerät (DG), wenn durch Änderungen an einer Hardware (HW) und/oder an einem Softwarecode (SW) des Produktivsystems (P) ein geändertes System (P') erzeugt wurde, wobei das Testsystem (400) zur Ausführung eines Verfahrens nach einem der vorstehenden Verfahrensansprüche bestimmt ist mit:
- Einer ersten Testeinheit (TE1), die ein Ausführen eines Testverfahrens (t) auf dem Produktivsystem (P) veranlasst und, die zum Erzeugen eines ersten Testergebnisses (T1) bestimmt ist;
- Einer zweiten Testeinheit (TE2), die ein Ausführen des Testverfahrens (t) auf dem geänderten System (P') veranlasst und, die zum Erzeugen eines zweiten Testergebnisses (T2) bestimmt ist;
- Einer Recheneinheit (RE), die zum Vergleichen des ersten Testergebnisses (T1) mit dem zweiten Testergebnis (T2) bestimmt ist, um eine spezifikationskonforme Funktionalität des geänderten Systems (P') zu prüfen;
- Einer Ausgabeschnittstelle (AS) zum Ausgeben eines Testergebnisses (TE).

14. Medizinisches Gerät (DG) mit einem Testsystem (400) oder mit Einheiten des Testsystems (400) nach dem unmittelbar vorangehenden Anspruch.

15. Medizinisches Behandlungssystem zur medizinischen Behandlung von Patienten mit einem medizinischen Gerät (DG) mit einem Produktivsystem (P) mit einer elektronischen Einheit, wobei die Funktionalitätsprüfung von Funktionen der elektronischen Einheit durch ein Verfahren nach einem der vorangehenden Verfahrensansprüche ausgeführt wird.
